# FASCICULE DE BREVET EUROPEEN

(11) **EP 0 709 356 B1**
(45) Date de publication et mention de la délivrance du brevet: **13.01.1999**
(21) Numéro de dépôt: 95402310.7
(22) Date de dépôt: 17.10.1995
(51) Int. Cl.: C07C 2/60, B01J 31/02

(54) **Composition catalytique et procédé pour l'alkylation d'hydrocarbures aliphatiques**
Katalytische Zusammenstellung und Verfahren für die Alkylierung von aliphatische Kohlenwasserstoffen
Catalytic composition and aliphatic hydrocarbon alkylation process

(30) Priorité: 24.10.1994 FR 9412778
(43) Date de publication de la demande: 01.05.1996
(73) Titulaire: INSTITUT FRANCAIS DU PETROLE, 92502 Rueil-Malmaison (FR)
(72) Inventeur: Chauvin, Yves, F-78230 Le Pecq (FR); Hirschauer, André, F-78360 Montesson (FR); Olivier, Hélène, F-92500 Rueil Malmaison (FR)

(56) Documents cités:
- EP-A- 0 488 073
- EP-A- 0 553 009
- EP-A- 0 576 323
- WO-A-91/16283

## Description

L'objet de la présente invention est une composition catalytique ionique au moins en partie liquide et un procédé utilisant cette composition pour la production d'hydrocarbures paraffiniques par réaction d'addition d'oléfines sur des isoparaffines en présence de catalyseurs de type Friedel-Craft modifiés.

On connait un grand nombre de catalyseurs acides, liquides ou solides susceptibles de réaliser l'alkylation des isoparaffines telles que l'isobutane ou l'isopentane, par des oléfines telles que le propylène, les butènes-1 et -2, l'isobutène. Les catalyseurs les plus couramment utilisés dans la pratique industrielle sont l'acide sulfurique concentré et l'acide fluorhydrique seul ou en mélange avec des acides de Lewis tels que le trifluorure de bore. Ces prodédés souffrent d'inconvénients majeurs: l'acide fluorhydrique en raison de sa toxicité et de sa forte volatilité; l'acide sulfurique en raison d'une consommation importante du catalyseur nécessitant un retraitement onéreux. C'est pourquoi on a préconisé l'emploi de catalyseurs solides ou supportés sur des solides tels que les aluminosilicates ou les oxydes métalliques tels que la zircone traitée par le l'acide sulfurique. Mais les catalyseurs solides se sont montrés généralement peu sélectifs et peu actifs. L'utilisation du chlorure d'aluminium a aussi été étudiée et proposée.

Il a été proposé dans les brevets français n° 2.626.572 et n° 2.692.888 d'utiliser pour catalyser la réaction d'alkylation paraffinique les complexes ioniques liquides que font les halogénures d'aluminium avec certains halogénures d'ammonium quaternaires ou avec certains halohydrates d'amines. Ces complexes, dits encore "sels fondus", ont été décrits par C.H. Hussey dans *Advances in Molten Salts Chemistry,* vol. 5, p185, Elsevier, New-York, 1985 et par C.A. Angell et J.W. Shuppert dans J. Phys. Chem. 84, 538, 1980. Ces catalyseurs sont particulièrement simples à mettre en oeuvre.

Dans ces brevets il a été montré que les catalyseurs les plus actifs sont obtenus par mélange d'un équivalent d'halogénure d'ammonium quaternaire ou d'halohydrate d'amine avec un équivalent et plus de trihalogénure d'aluminium

Il a maintenant été trouvé que l'addition de composés de cuivre (I) et notamment d'halogénures cuivreux aux sels précédents améliorait la sélectivité et la stabilité du catalyseur, permettait d'éviter l'introduction de protons additionnels pour réactiver le catalyseur et diminuait la teneur en chlore des alkylats qui sont les produits de réaction.

Plus précisément l'objet de l'invention est une composition catalytique comprenant au moins un halogénure d'aluminium, au moins un halogénure d'ammonium quaternaire et/ou au moins un halohydrate d'amine et au moins un composé cuivreux. Un autre objet de l'invention est un procédé pour l'alkylation d'au moins une isoparaffine par au moins une oléfine, procédé dans lequel les paraffines et les oléfines sont mises en contact avec la dite composition catalytique.

Les halogénures d'aluminium utilisables dans le cadre de l'invention sont de préférence le chlorure d'aluminium et le bromure d'aluminium. Les halogénures d'hydrocarbylalumium sont déconseillés.

Les halogénures d'ammonium quaternaires utilisables dans le cadre de l'invention sont ceux décrits dans le brevet français 2.626.572. Les sels d'ammonium quaternaires, acycliques ou faisant partie d'un cycle répondent aux formules générales suivantes:

R¹R²R³R⁴N⁺ X⁻ (I)

R¹R²N⁺=CR³R⁴⁺ X⁻ (II)

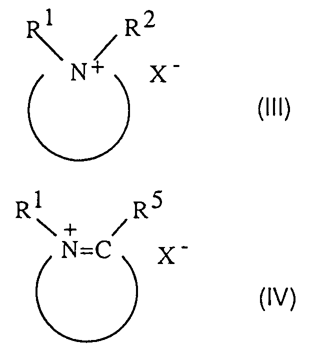

dans lesquelles R¹, R², R³, R⁴ ou R⁵ , identiques ou différents, représentent chacun des restes hydrocarbyles comprenant généralement 1 à 12 atomes de carbone, par exemple alkyl, cycloalkyl, aryl, aralkyl, R⁵ pouvant être également l'hydrogène ou des restes hydrocarbyles substitués comprenant par exemple d'autres atomes comme l'azote. R¹, R², R³, ou R⁴ peuvent également être l'hydrogène, à l'exception du cation NH4+ et de préférence un seul substituant (R¹, R², R³, R⁴ ou R⁵) peut représenter l'hydrogène. Des radicaux tels que R⁶ peuvent unir deux molécules telles que ci-dessus comme, par exemple R¹ R²N⁺=CR³-R⁶-CR³=N⁺R¹R² (X⁻)₂, R⁶ pouvant être un reste alkylène ou encore phénylène, les radicaux R¹, R², R³ étant définis comme précédemment.
Les cycles III et IV sont constitués de 4 à 10 atomes, de préférence de 5 à 6 atomes qui peuvent comprendre, outre l'azote de l'ammonium quaternaire, des atomes de carbone ou éventuellement d'autres atomes d'azote, 1 ou 2 généralement.
Parmi les groupements R¹, R², R³, R⁴ ou R⁵ on mentionnera les radicaux méthyl, éthyl, propyl, isopropyl, butyl, secondaire butyl, tertiaire butyl, amyl, méthylène, éthylidène, phényl ou benzyl; R⁶ pourra être un groupement méthylène, éthylène, propylène ou phénylène.
Dans ces formules X représente un ion halogènure, par exemple l'ion bromure ou l'ion chlorure.
A titre d'exemples de sels d'ammonium utilisables selon l'invention on peut citer plus particulièrement les sels d'imidazolium et de pyridinium tels que le chlorure de N-butylpyridinium, le bromure d'éthylpyridinium, le chlorure de butyl-3 méthyl-1 imidazolium, le chlorure de diéthylpyrazolium, le chlorure d'éthyl-3 méthyl-1 imidazolium.

Les halohydrates d'amines et notamment chlorhydrates ou les bromhydrates d'amines plus particulièrement utilisables dans le cadre de l'invention comportent de préférence une mole d'acide halohydrique (chlorhydrique ou bromhydrique préférés) par mole d'amine mais peuvent également comporter deux moles d'acide par mole d'amine. On peut aussi utiliser des mélanges d'halohydrates à une mole et à deux moles d'acide halohydrique. Les halohydrates dérivent d'amines ou de diamines acycliques ou d'amines faisant partie d'un cycle qui peut comporter un ou plusieurs atomes d'azote et répondant aux formules générales suivantes: 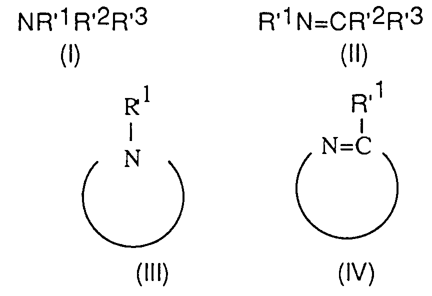 dans lesquelles R'¹ ,R'²,R'³ identiques ou différents, représentent des restes hydrocarbyles comportant généralement 1 à 12 atomes de carbone par exemple alkyl, cycloalkyl, aryl, aralkyl. L'un de ces substituants R'¹, R'², R'³ peut être l'hydrogène.Les cycles III et IV sont généralement constitués de 4 à 10 atomes, de préférence de 5 à 6 atomes qui peuvent comprendre, outre un ou des atomes d'azote, des atomes de carbone liés par des liaisons simples ou doubles. Les cycles III et IV peuvent être condensés avec d'autres cycles et porter des substituants tels que definis ci-dessus, des fonctions amines, des atomes de fluor, de chlore, de brome.

Parmi les groupements R'¹, R'² et R'³ on mentionnera les radicaux méthyl, éthyl, propyl, isopropyl, butyl, sec-butyl, amyl, méthylène, éthylidène, phényl, benzyl. Les cycles tels que IV sont représentés par les familles des pyridines, des imidazoles, des triazines, des pyrazoles, des pyrimidines, des triazoles.

Les halohydrates préférés sont les chlorhydrates ou les bromhydrates de la pyridine, des picolines-2, -3 ou -4, des lutidines, de l'éthyl-2pyridine, de l'isopropyl-3 pyridine, des chloro-2 ou-4 pyridine, de la N,N-diméthylamino-4 pyridine, du N-méthylimidazole, du N-butylimidazole, de la pipéridine, de la N-méthylimidazoline.

Un moyen essentiel déterminant selon l'invention est la présence du cuivre (I).
Les composés du cuivre(l) utilisables dans le cadre de l'invention sont par exemple l'acétate, le sulfate, le nitrate, le perchlorate et le composé Cᵤ⁺AlCl₄⁻. Très avantageusement c'est un halogénure, ce qui évite l'introduction d'ions supplémentaires dans le milieu réactionnel.

Les halogénures de cuivre préférés dans le cadre de l'invention sont le chlorure cuivreux et le bromure cuivreux.

On peut également introduire comme produit de départ un composé cuivrique, mais il se transforme dans le milieu réactionnel, au moins en partie, en un composé cuivreux.

Les composants des mélanges tels que définis ci-dessus sont, de préférence, mis en oeuvre dans des rapports molaires halogénure d'aluminium:halogénure d'ammonium quaternaire et/ou halohydrate d'amine compris entre 1:1,1 et 1:3,5, de préférence entre 1:2 et 1:3 ; halogénure d'aluminium:halogénure cuivreux compris entre 1:0,1 et 1:1, de préférence entre 1:0,2 et 1:0,5.

Les composés entrant dans la composition selon l'invention, peuvent être mélangés dans un ordre quelconque, à une température comprise entre -20°C et + 80°C. Le mélange peut se faire par une simple mise en contact suivie d'une agitation jusqu'à formation d'un liquide ou d'une suspension manipulable. Ce mélange peut se faire en dehors du réacteur d'alkylation ou encore dans ce réacteur, en présence ou en absence d'hydrocarbures. On peut avantageusement faire le mélange en présence d'un composé organique polaire liquide, peu basique et suffisamment volatil qui soit miscible avec le mélange des composants du mélange et qui sera ensuite éliminé par évaporation avant la réaction d'alkylation. Les composés utilisables selon l'invention sont, par exemple, les ethers et les nitriles et parmi ces derniers plus particulièrement l'acétonitrile ou le propionitrile. De cette façon on obtient une préparation homogène, (et non plus une suspension) facilement manipulable.

L'invention a également pour objet une composition catalytique avec le mélange des composants tel que défini précédemment, qui compte également au moins un composé organique, solvant du mélange des composants, tel que défini ci-dessus.

L'invention a également pour objet un procédé d'alkylation d'au moins une isoparaffine par au moins une oléfine en présence d'une composition catalytique définie précédemment.

Les paraffines susceptibles d'être alkylées selon l'invention sont plus particulièrement l'isobutane, l'isopentane, le méthyl-2 butane, les méthyl-2 et méthyl-3-pentanes. Les oléfines utilisables selon l'invention sont, plus particulièrement , l'éthylène, le propylène, les n-butènes, l'isobutène, les n-pentènes et iso pentènes, et de façon plus générale les oléfines ayant 2 à 7 atomes de carbone. L'invention est ainsi avantageuse pour traiter un effluent en C₆ issu de la dimérisation de coupe C₃ oléfinique.

La réaction catalytique d'alkylation est conduite en phase hydrocarbonée liquide, en système fermé, en système semi-ouvert ou en continu avec un étage de réaction qui est la pratique courante en alkylation aliphatique. L'isoparaffine et l'oléfine peuvent être introduites séparément ou en mélange. Dans un système en continu, ou semi-continu, le rapport molaire entre l'isoparaffine et l'oléfine est par exemple compris entre 2 et 100, et avantageusement entre 10 et 50, de préférence entre 5 et 20. Dans un système semi-ouvert on introduira d'abord de l'isoparaffine puis de l'oléfine ou un mélange d'isoparaffine et d'oléfine. Une vigoureuse agitation doit assurer un bon contact entre les réactifs et le mélange catalytique. La température de réaction peut être comprise entre -40°C et +70°C, de préférence entre -20°C et +30°C. La pression peut être comprise entre la pression atmosphérique et 10 MPa, mais sera suffisante pour maintenir les réactifs en phase liquide. La chaleur engendrée par la réaction peut être éliminée par tous les moyens connus de l'homme de l'art. A la sortie du réacteur la phase hydrocarbonée est séparée de la phase ionique par décantation, puis les hydrocarbures sont séparés par distillation et l'isoparaffine de départ qui n'a pas été convertie est recyclée au réacteur,

Il a été observé, que, lorsque le catalyseur a perdu une partie notable de son activité, on peut le réactiver en ajoutant au moins un additif électrophile, par exemple un halogénure d'aluminium, un acide halohydrique, un halogénure d'alkyle.

Les quantités d'additif ajoutées sont de préférence telles que le rapport molaire halogénure d'aluminium : additif soit compris entre 0,001 et 1 et avantageusement entre 0,001 et 0,1.

Les exemples suivants illustrent l'invention sans en imiter la portée.

### EXEMPLE N°1

### Préparation de l'organochloroaluminate.

Dans un ballon en verre muni d'un agitateur magnétique, purgé d'air et d'humidité et maintenu à 10°C on a introduit successivement 22,9 g de chlorure d'aluminium fraîchement distillé, 20 mL d'heptane et par portions 16,2 g de chlorure de butyl-1 méthyl-3 imidazolium. On obtient ainsi une composition liquide.

### Alkylation de l'isobutane par le butène-2.

7 mL (9,5 g) de la composition précédente sont introduits dans un réacteur en verre destiné à la réaction d'alkylation dans lequel on ajoute 1,9 g de chlorure cuivreux anhydre. On obtient ainsi une suspension visqueuse qui peut néanmoins être agitée vigoureusement à l'aide d'un barreau magnétique. On introduit sur cette suspension 40 g d'isobutane et 8,3 g de butane (étalon interne). Puis on injecte en continu un mélange isobutane/n-butane/butène-2 contenant 40% en poids de butène à la vitesse de 0,3 volume de butène -2 liquide par volume de composition catalytique et par heure (V/V/H = 0,3). On arrête la réaction lorsque la conversion de l'isobutane est de 15 à 30% suivant les essais (c'est-à-dire de 5 à 10 heures de réaction). On soutire la phase hydrocarbonée et on recommence comme ci-avant. On a refait la réaction 16 fois de suite. L'analyse du mélange d'hydrocarbures est effectuée par chromatographie en phase vapeur. La conversion du butène-2 était toujours quantitative. La composition moyenne de l'alkylat, les valeurs calculées des nombres d'octane "Recherche" et "Moteur" et la teneur en chlore organique sont données dans le tableau 1.

### EXEMPLE COMPARATIF (sans halogénure cuivreux)

On a opéré dans des conditions identiques à celles de l'exemple 1, mais en absence de chlorure cuivreux. Après 5 essais successifs on a constaté que la conversion du butène-2 n'était plus totale et que la sélectivité vers les composés à haut indice d'octane décroissait. La composition moyenne de l'alkylat, les valeurs calculées des nombres d'octane "Recherche" et "Moteur" et la teneur en chlore organique sont données dans le tableau 1.

### EXEMPLE N° 2

### Préparation de la composition catalytique

Dans un ballon en verre muni d'un agitateur magnétique, purgé d'air et d'humidité et maintenu à 10°C on a introduit successivement 22,9 g de chlorure d'aluminium fraîchement distillé, 20 mL d'heptane et par portions 16,2 g de chlorure de butyl-1 méthyl-3 imidazolium. On obtient ainsi une composition liquide qu'on introduit dans un autre ballon contenant 7,5 g de chlorure cuivreux anhydre. A la suspension ainsi obtenue on ajoute 20 mL d'acétonitrile sec ce qui conduit à un liquide homogène. On évapore l'acétonitrile. Le mélange devient alors visqueux. On y ajoute 9 mL d'un chloroaluminate identique à celui qui a servi à la préparation précédente. On obtient ainsi un liquide homogène qui a été utilisé pour l'alkylation.

### Alkylation de l'isobutane par le butène-2.

On a opéré comme dans l'exemple 1. Les résultats qui ont été obtenus sont comparables: NOR (calculé): 98,2; NOM (calculé): 96,1.

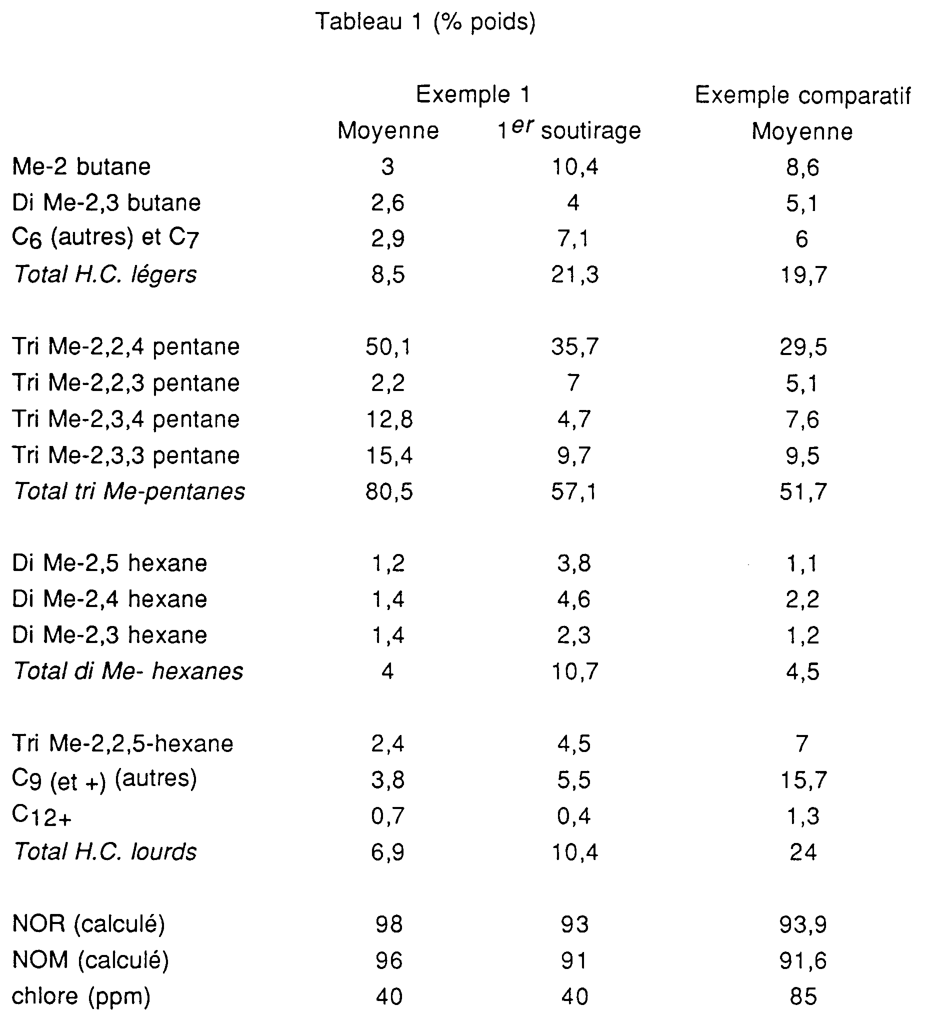

## Revendications

1. Composition catalytique comprenant au moins un halogénure d'aluminium, au moins un halogénure d'ammonium quaternaire et/ou au moins un halohydrate d'amine, et au moins un composé cuivreux.

2. Composition catalytique selon la revendication 1, caractérisée en ce que le composé cuivreux est un halogénure cuivreux.

3. Composition catalytique selon l'une des revendications précédentes dans laquelle l'halogénure cuivreux est choisi dans le groupe constitué par le chlorure de cuivre(l) et le bromure de cuivre(l).

4. Composition catalytique selon l'une des revendications précédentes dans laquelle le composé cuivreux est choisi dans le groupe formé par l'acétate, le sulfate, le perchlorate et le nitrate cuivreux, et le composé Cᵤ+AlCl₄⁻.

5. Composition catalytique selon l'une des revendications précédentes dans laquelle l'halogénure d'aluminium est choisi dans le groupe constitué par le chlorure d'aluminium et le bromure d'aluminium.

6. Composition catalytique selon l'une des revendications précédentes dans laquelle l'halogénure d'ammonium quaternaire est choisi dans le groupe formé par les composés de formule générale:
R¹R²R³R⁴N⁺ X⁻
R¹R²N=CR³R⁴⁺ X⁻ 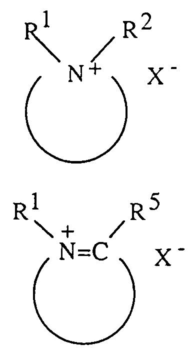
dans lesquelles R¹,R²,R³,R⁴,R⁵ identiques ou différents représentent des restes hydrocarbyles ayant 1 à 12 atomes de carbone, R⁵ pouvant être également l'hydrogène, et dans lesquelles les cycles sont constitués de 4 à 10 atomes, X représentant l'ion halogénure.

7. Composition catalytique selon l'une des revendications 1 à 5 dans laquelle l'halogénure d'ammonium quaternaire est choisi dans le groupe formé par les composés de formule générale:
R¹R²R³R⁴N⁺ X⁻
R¹R²N=CR³R⁴⁺ X⁻ 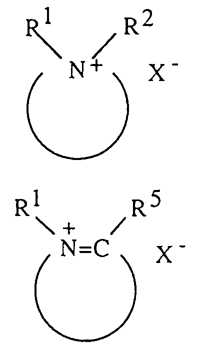
dans lesquelles R¹,R²,R³,R⁴,R⁵ représentent l'hydrogène, a l'exception de NH4⁺.

8. Composition catalytique selon la revendication 7, dans laquelle un seul substituant R1, R2, R3, R4, R5 représente l'hydrogène.

9. Composition catalytique selon l'une des revendications précédentes à dans laquelle l'halogénure d'ammonium quaternaire a pour formule générale:
R¹R²⁺N=CR³-R⁶-R³C=N⁺R¹R² (X⁻)₂
dans laquelle R¹,R²,R³ identiques ou différents représentent des restes hydrocarbyles ayant 1 à 12 atomes de carbone, et R⁶ représente un reste alkylène ou phénylène, X représentant l'ion halogénure.

10. Composition catalytique selon l'une des revendications 1 à 5 dans laquelle l'halogénure d'ammonium quaternaire a pour formule générale:
R¹R²⁺N=CR³-R⁶-R³C=N⁺R¹R² (X⁻)₂
dans laquelle R¹,R²,R³ représentent l'hydrogène.

11. Composition catalytique selon la revendication 10, dans laquelle un seul substitutant R¹, R², R³ représente l'hydrogène.

12. Composition catalytique selon l'une des revendications précédentes dans laquelle l'halogénure d'ammonium quaternaire est choisi dans le groupe constitué par le chlorure de N-butylpyridinium, le bromure d'éthylpyridinium, le chlorure de butyl-3 méthyl-1 imidazolium, le chlorure de diéthylpyrazolium, le chlorure d'éthyl-3 méthyl-1 imidazolium.

13. Composition catalytique selon l'une des revendication précédentes dans laquelle l'halohydrate d'amine est choisi dans le groupe constitué par les halohydrates comportant une mole d'acide halohydrique par mole d'amine et les halohydrates comportant 2 moles d'acide halohydrique par mole d'amine.

14. Composition catalytique selon l'une des revendications précédentes dans laquelle l'halohydrate d'amine dérive de l'amine choisie dans le groupe des composés de formules générales
NR'¹R'²R'³
R'¹N=CR'²R'³ 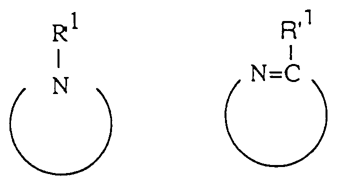
dans lesquelles R'¹ ,R'²,R'³, identiques ou différents représentent des restes hydrocarbyles ayant 1 à 12 atomes de carbone, et dans lesquelles les cycles sont constitués de 4 à 10 atomes, l'un des substituants R'¹, R'² ou R'³ pouvant être l'hydrogène.

15. Composition catalytique selon l'une des revendications précédentes dans laquelle l'acide halohydrique est choisi dans le groupe formé par l'acide chlorhydrique et l'acide bromhydrique.

16. Composition catalytique selon l'une des revendications précédentes dans laquelle les halohydrates sont choisis dans le groupe des chlorhydrates et des bromhydrates de la pyridine, des picolines-2, -3 et- 4, du N-méthylimidazole, du N-butylimidazole, des lutidines, de l'éthyl-2 pyridine, de l'isopropyl-3 pyridine, des chloro-2 ou -4 pyridine, de la N,N-diméthylamino-4 pyridine, de la pipéridine, de la N-méthylimidazoline.

17. Composition catalytique selon l'une des revendications précédentes dans laquelle le rapport molaire entre l'halogénure d'aluminium et l'halogénure d'ammonium quaternaire et/ou l'halohydrate d'amine est compris entre 1:1,1 et 1:3,5.

18. Composition catalytique selon l'une des revendications précédentes dans laquelle le rapport molaire entre l'halogénure d'aluminium et l'halogénure d'ammonium quaternaire et/ou l'halohydrate d'amine est compris entre 1:2 et 1:3 .

19. Composition catalytique selon l'une des revendications précédentes dans laquelle le rapport molaire entre l'halogénure d'aluminium et l'halogénure cuivreux est compris entre 1:0,1 et 1:1.

20. Composition catalytique selon l'une des revendications précédentes dans laquelle le rapport molaire entre l'halogénure d'aluminium et l'halogénure cuivreux est compris entre 1:0,2 et 1:0,5.

21. Composition catalytique selon l'une des revendications précédentes comportant également au moins un composé organique polaire liquide, peu basique, miscible avec le mélange des composants.

22. Composition selon l'une des revendications précédentes dans laquelle le composé organique polaire est choisi dans le groupe formé par les éthers et les nitriles.

23. Composition catalytique selon la revendication précédente dans laquelle le composé organique polaire est choisi dans le groupe formé par l'acétonitrile et le propionitrile..

24. Composition catalytique selon l'une des revendications précédentes, comportant également au moins un additif électrophile.

25. Composition catalytique selon l'une des revendications précédentes, comportant également au moins un additif choisi dans le groupe formé par un halogénure d'aluminium, un acide halohydrique, un halogénure d'alkyle.

26. Composition catalytique selon l'une des revendications 24 à 25, dans laquelle le rapport molaire halogènure d'aluminium : additif est compris entre 0,001 et 1.

27. Composition catalytique selon l'une des revendications 24 à 25 dans laquelle le rapport molaire halogènure d'aluminium : additif est compris entre 0,001 et 0,1.

28. Procédé pour l'alkylation d'au moins une isoparaffine par au moins une oléfine en présence d'une composition catalytique selon l'une au moins des revendications 1 à 27.

29. Procédé selon la revendication 28, lequel l'oléfine comporte 2 à 7 atomes de carbone.

30. Procédé selon l'une des revendications 28 à 30 dans lequel l'isoparaffine est choisie dans le groupe formé par l'isobutane, le méthyl-2 butane, les méthyl-2 et méthyl-3 pentanes, l'isopentane.

31. Procédé selon l'une des revendications 28 à 30 dans lequel l'oléfine est choisie dans le groupe formé par l'éthylène, le propylène, les n-butènes, l'isobutène, les n-pentènes et les isopentènes.

32. Procédé selon l'une des revendications 28 à 31 dans lequel la réaction est conduite de façon continue ou semi continue avec un rapport molaire entre la paraffine et l'oléfine est compris entre 2 et 100.

33. Procédé selon l'une des revendications 28 à 32 dans lequel la réaction est conduite avec un rapport molaire entre la paraffine et l'oléfine de 5 à 20.

34. Procédé selon l'une des revendications 28 à 33, dans lequel il est ajouté au moins un additif electrophile.

35. Procédé selon l'une des revendications 28 à 34, dans lequel il est ajouté au moins un additif choisi dans le groupe formé par un halogénure d'aluminium, un acide halohydrique, un halogénure d'alkyle.

36. Procédé selon l'une des revendications 34 à 35, dans lequel le rapport molaire halogénure d'ammonium : additif est compris entre 0,001 et 1.

37. Procédé selon l'une des revendications 34 à 35, dans lequel le rapport molaire halogénure d'ammonium : additif est compris entre 0,001 et 0,1.

38. Procédé de préparation d'une composition catalytique selon l'une des revendications 1 à 27 dans lequel au moins un halogénure d'aluminium, au moins un halogénure d'ammonium quaternaire et/ou au moins un halohydrate d'amine et au moins un composé cuivreux sont mis en contact à une température comprise entre -20°C et +80°C en présence d'un composé organique polaire liquide, peu basique et miscible avec le mélange des composants.

## Claims

1. A catalytic composition comprising at least one aluminium halide, at least one quaternary ammonium halide and/or at least one amine halohydrate, and at least one cuprous compound.

2. A catalytic composition according to claim 1 characterised in that the cuprous compound is a cuprous halide.

3. A catalytic composition according to one of the preceding claims wherein the cuprous halide is selected from the group formed by copper (I) chloride and copper (I) bromide.

4. A catalytic composition according to one of the preceding claims wherein the cuprous compound is selected from the group formed by cuprous acetate, sulphate, perchlorate and nitrate and the compound Cᵤ+AlCl₄-.

5. A catalytic composition according to one of the preceding claims wherein the aluminium halide is selected from the group formed by aluminium chloride and aluminium bromide.

6. A catalytic composition according to one of the preceding claims wherein the quaternary ammonium halide is selected from the group formed by compounds of the general formula:
R¹R²R³R⁴N⁺ X⁻
R¹R²N=CR³R⁴⁺ X⁻ 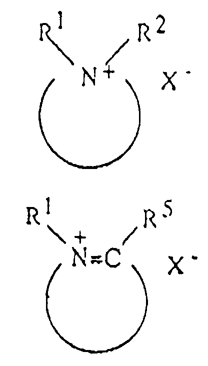
in which R¹, R², R³, R⁴ and R⁵ which are identical or different represent hydrocarbyl residues having from 1 to 12 carbon atoms, wherein R⁵ may also be hydrogen, and in which the rings are formed by from 4 to 10 atoms, X representing the halide ion.

7. A catalytic composition according to one of claims 1 to 5 wherein the quaternary ammonium halide is selected from the group formed by the compounds of the general formula:
R¹R²R³R⁴N⁺ X⁻
R¹R²N=CR³R⁴⁺ X⁻ 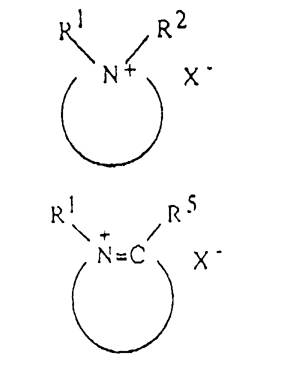
in which R¹, R², R³, R⁴ and R⁵ represent hydrogen, except for NH₄+.

8. A catalytic composition according to claim 7 wherein a single substituent R¹, R², R³, R⁴ and R⁵ represents hydrogen.

9. A catalytic composition according to one of the preceding claims wherein the quaternary ammonium halide is of the following general formula:
R¹R²+N=CR³-R⁶-R³C=N+R¹R²(X⁻)₂
in which R¹, R² and R³ which are identical or different represent hydrocarbyl residues having from 1 to 12 carbon atoms and R⁶ represents an alkylene or phenylene residue, X representing the halide ion.

10. A catalytic composition according to one of claims 1 to 5 wherein the quaternary ammonium halide is of the following general formula:
R¹R²+N=CR³-R⁶-R³C=N+R¹R²(X⁻)₂
in which R¹, R² and R³ represent hydrogen.

11. A catalytic composition according to claim 10 wherein a single substituent R¹, R² and R³ represents hydrogen.

12. A catalytic composition according to one of the preceding claims wherein the quaternary ammonium halide is selected from the group formed by N-butylpyridinium chloride, ethylpyridinium bromide, 3-butyl 1-methyl imidazolium chloride, diethylpyrazolium chloride and 3-ethyl 1-methyl imidazolium chloride.

13. A catalytic composition according to one of the preceding claims wherein the amine halohydrate is selected from the group formed by halohydrates comprising one mole of halohydric acid per mole of amine and the halohydrates comprising 2 moles of halohydric per mole of amine.

14. A catalytic composition according to one of the preceding claims wherein the amine halohydrate derives from the amine selected from the group of compounds of the following general formulae:
NR'¹R'²R'³
R'¹N=CR'²R'³ 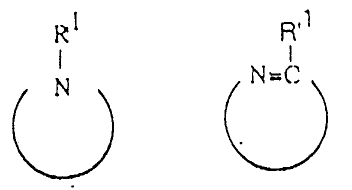
in which R'¹ R'² and R'³ which are identical or different represent hydrocarbyl residues having from 1 to 12 carbon atoms and in which the rings are formed by from 4 to 10 atoms, wherein one of the substituents R'¹, R'² and R'³ may be hydrogen.

15. A catalytic composition according to one of the preceding claims wherein the halohydric acid is selected from the group formed by hydrochloric acid and hydrobromic acid.

16. A catalytic composition according to one of the preceding claims wherein the halohydrates are selected from the group of chlorohydrates and bromohydrates of pyridine, 2-, 3- and 4-picolines, N-methylimidazole, N-butylimidazole, lutidines, 2-ethyl pyridine, 3-isopropyl pyridine, 2-or 4-chloropyridine, N,N-dimethyl-4-amino pyridine, piperidine and N-methylimidazoline.

17. A catalytic composition according to one of the preceding claims wherein the molar ratio between the aluminium halide and the quaternary ammonium halide and/or the amine halohydrate is between 1:1.1 and 1:3.5.

18. A catalytic composition according to one of the preceding claims wherein the molar ratio between the aluminium halide and the quaternary ammonium halide and/or the amine halohydrate is between 1:2 and 1:3.

19. A catalytic composition according to one of the preceding claims wherein the molar ratio between the aluminium halide and the cuprous halide is between 1:0.1 and 1:1.

20. A catalytic composition according to one of the preceding claims wherein the molar ratio between the aluminium halide and the cuprous halide is between 1:0.2 and 1:0.5.

21. A catalytic composition according to one of the preceding claims also comprising at least one organic polar liquid compound of low basicity which is miscible with the mixture of the components.

22. A composition according to one of the preceding claims wherein the organic polar compound is selected from the group formed by ethers and nitriles.

23. A catalytic composition according to the preceding claim wherein the organic polar compound is selected from the group formed by acetonitrile and propionitrile.

24. A catalytic composition according to one of the preceding claims also comprising at least one electrophile additive.

25. A catalytic composition according to one of the preceding claims also comprising at least one additive selected from the group formed by an aluminium halide, a halohydric acid and an alkyl halide.

26. A catalytic composition according to one of claims 24 and 25 wherein the aluminium halide: additive molar ratio is between 0.001 and 1.

27. A catalytic composition according to one of claims 24 and 25 wherein the aluminium halide: additive molar ratio is between 0.001 and 0.1.

28. A process for the alkylation of at least one isoparaffin by at least one olefin in the presence of a catalytic composition according to one at least of claims 1 to 27.

29. A process according to claim 28 wherein the olefin comprises from 2 to 7 carbon atoms.

30. A process according to one of claims 28 to 30 wherein the isoparaffin is selected from the group formed by isobutane, 2-methyl butane, 2-methyl and 3-methyl pentanes and isopentane.

31. A process according to one of claims 28 to 30 wherein the olefin is selected from the group formed by ethylene, propylene, n-butenes, isobutene, n-pentenes and isopentenes.

32. A process according to one of claims 28 to 31 wherein the reaction is effected continuously or semi-continuously with a molar ratio between the paraffin and the olefin of between 2 and 100.

33. A process according to one of claims 28 to 32 wherein the reaction is effected with a molar ratio between the paraffin and the olefin of from 5 to 20.

34. A process according to one of claims 28 to 33 wherein at least one electrophile additive is added.

35. A process according to one of claims 28 to 34 wherein there is added at least one additive selected from the group formed by an aluminium halide, a halohydric acid and an alkyl halide.

36. A process according to one of claims 34 and 35 wherein the ammonium halide:additive molar ratio is between 0.001 and 1.

37. A process according to one of claims 34 and 35 wherein the ammonium halide:additive molar ratio is between 0.001 and 0.1.

38. A process for the preparation of a catalytic composition according to one of claims 1 to 27 wherein at least one aluminium halide, at least one quaternary ammonium halide and/or at least one amine halohydrate and at least one cuprous compound are brought into contact at a temperature of between -20°C and +80°C in the presence of an organic polar liquid compound which is of low basicity and which is miscible with the mixture of the components.

## Patentansprüche

1. Katalytische Zusammensetzung, die mindestens ein Aluminiumhalogenid, mindestens ein quaternäres Ammoniumhalogenid und/oder mindestens ein Aminhydrohalogenid sowie mindestens eine Kupfer(I)-Verbindung umfaßt.

2. Katalytische Zusammensetzung nach Anspruch 1, dadurch gekennzeichnet, daß die Kupfer(I)-Verbindung ein Kupfer(I)-halogenid ist.

3. Katalytische Zusammensetzung nach einem der vorhergehenden Ansprüche, in der das Kupfer(I)-halogenid ausgewählt wird aus der Gruppe, die besteht aus Kupfer(I)-chlorid und Kupfer(I)-bromid.

4. Katalytische Zusammensetzung nach einem der vorhergehenden Ansprüche, in der die Kupfer(I)-Verbindung ausgewählt wird aus der Gruppe, die besteht aus Kupfer(I)-acetat, -sulfat, -perchlorat und -nitrat und der Verbindung Cu⁺AlCl₄⁻.

5. Katalytische Zusammensetzung nach einem der vorhergehenden Ansprüche, in der das Aluminiumhalogenid ausgewählt wird aus der Gruppe, die besteht aus Aluminiumchlorid und Aluminiumbromid.

6. Katalytische Zusammensetzung nach einem der vorhergehenden Ansprüche, in der das quaternäre Ammoniumhalogenid ausgewählt wird aus der Gruppe, die besteht aus Verbindungen der allgemeinen Formel
R¹R²R³R⁴N⁺ X⁻
R¹R²N=CR³R⁴N⁺ X⁻ 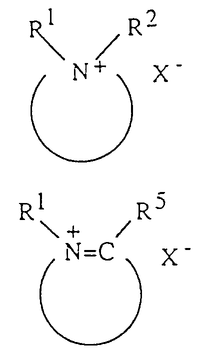
worin R¹, R², R³, R⁴ und R⁵, die gleich oder verschieden sind, Hydrocarbyl-Reste mit 1 bis 12 Kohlenstoffatomen darstellen, wobei R⁵ auch für Wasserstoff stehen kann, und in denen die Cyclen aus 4 bis 10 Atomen bestehen und X ein Halogenidion darstellt.

7. Katalytische Zusammensetzung nach einem der Ansprüche 1 bis 5, in der das quaternäre Ammoniumhalogenid ausgewählt wird aus der Gruppe, die besteht aus Verbindungen der allgemeinen Formel
R¹R²R³R⁴N⁺ X⁻
R¹R²N=CR³R⁴⁺ X⁻ 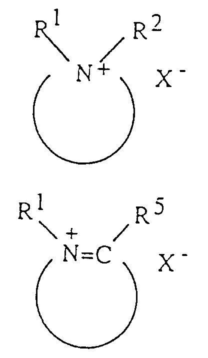
worin R¹, R², R³, R⁴ und R⁵ Wasserstoff bedeuten mit Ausnahme von NH₄⁺.

8. Katalytische Zusammensetzung nach Anspruch 7, in der nur einer der Substituenten R¹, R², R³, R⁴ und R⁵ für Wasserstoff steht.

9. Katalytische Zusammensetzung nach einem der vorhergehenden Ansprüche, in der das quaternäre Ammoniumhalogenid die allgemeine Formel hat:
R¹R²N⁺=CR³-R⁶-R³C=N⁺R¹R² (X⁻)₂
in der R¹, R² und R³, die gleich oder verschieden sind, Hydrocarbylreste mit 1 bis 12 Kohlenstoffatomen darstellen, R⁶ einen Alkylen- oder Phenylen-Rest darstellt und X ein Halogenidion bedeutet.

10. Katalytische Zusammensetzung nach einem der Ansprüche 1 bis 5, in der das quaternäre Ammoniumhalogenid die allgemeine Formel hat
R¹R²N⁺=CR³-R⁶-R³C=N⁺R¹R² (X⁻)₂
in der R¹, R² und R³ für Wasserstoff stehen.

11. Katalytische Zusammensetzung nach Anspruch 10, in der nur einer der Substituenten R¹, R² und R³ für Wasserstoff steht.

12. Katalytische Zusammensetzung nach einem der vorhergehenden Ansprüche, in der das quaternäre Ammoniumhalogenid ausgewählt wird aus der Gruppe, die besteht aus N-Butylpyridiniumchlorid, Ethylpyridiniumbromid, 3-Butyl-1-methylimidazoliumchlorid, Diethylpyrazoliumchlorid und 3-Ethyl-1-methyl-imidazoliumchlorid.

13. Katalytische Zusammensetzung nach einem der vorhergehenden Ansprüche, in der das Aminhydrohalogenid ausgewählt wird aus der Gruppe, die besteht aus Hydrohalogeniden, die 1 mol Halogenwasserstoffsäure pro mol Amin enthalten, und Hydrohalogeniden, die 2 mol Halogenwasserstoffsäure pro mol Amin enthalten.

14. Katalytische Zusammensetzung nach einem der vorhergehenden Ansprüche, in der das Aminhydrohalogenid von einem Amin abgeleitet ist, das ausgewählt wird aus der Gruppe der Verbindungen der allgemeinen Formeln:
NR'¹R'²R'3
R'¹N-CR'²R'³ 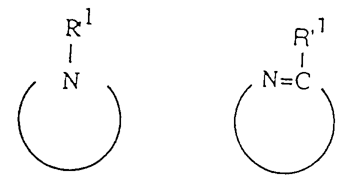
worin R'¹, R'² und R'³, die gleich oder verschieden sind, Hydrocarbylreste mit 1 bis 12 Kohlenstoffatomen bedeuten und in denen die Cyclen aus 4 bis 10 Atomen bestehen, wobei einer der Substituenten R'¹, R'² oder R'³ Wasserstoff sein kann.

15. Katalytische Zusammensetzung nach einem der vorhergehenden Ansprüche, in der die Halogenwasserstoffsäure ausgewählt wird aus der Gruppe, die besteht aus Chlorwasserstoffsäure und Bromwasserstoffsäure.

16. Katalytische Zusammensetzung nach einem der vorhergehenden Ansprüche, in der die Hydrohalogenide ausgewählt werden aus der Gruppe, die besteht aus Hydrochloriden und Hydrobromiden von Pyridin, 2-, 3- und 4-Picolinen, N-Methylimidazol, N-Butylimidazol, Lutidinen, 2-Ethyl-pyridin, 3-Isopropyl-pyridin, 2-Chlor- oder 4-Chlor-pyridin, 4-N,N-Dimethylamino-pyridin, Piperidin und N-Methylimidazolin.

17. Katalytische Zusammensetzung nach einem der vorhergehenden Ansprüche, in der das Molverhältnis von Aluminiumhalogenid zu quaternärem Ammoniumhalogenid und/oder Aminhydrochlorid zwischen 1:1,1 und 1:3,5 liegt.

18. Katalytische Zusammensetzung nach einem der vorhergehenden Ansprüche, in der das Molverhältnis von Aluminiumhalogenid zu quaternärem Ammoniumhalogenid und/oder Aminhydrohalogenid zwischen 1:2 und 1:3 liegt.

19. Katalytische Zusammensetzung nach einem der vorhergehenden Ansprüche, in der das Molverhältnis von Aluminiumhalogenid zu Kupfer(I)halogenid zwischen 1:0,1 und 1:1 liegt.

20. Katalytische Zusammensetzung nach einem der vorhergehenden Ansprüche, in der das Molverhältnis von Aluminiumhalogenid zu Kupfer(I)-halogenid zwischen 1:0,2 und 1:0,5 liegt.

21. Katalytische Zusammensetzung nach einem der vorhergehenden Ansprüche,die außerdem mindestens eine flüssige polare organische Verbindung enthält, die wenig basisch und mit dem Komponenten-Gemisch mischbar ist.

22. Zusammensetzung nach einem der vorhergehenden Ansprüche, in der die polare organische Verbindung ausgewählt wird aus der Gruppe, die besteht aus Ethern und Nitrilen.

23. Katalytische Zusammensetzung nach dem vorhergehenden Anspruch, in der die polare organische Verbindung ausgewählt wird aus der Gruppe, die besteht aus Acetonitril und Propionitril.

24. Katalytische Zusammensetzung nach einem der vorhergehenden Ansprüche, die außerdem mindestens ein elektrophiles Additiv enthält.

25. Katalytische Zusammensetzung nach einem der vorhergehenden Ansprüche, die außerdem mindestens ein Additiv enthält, das ausgewählt wird aus der Gruppe, die besteht aus einem Aluminiumhalogenid, einer Halogenwasserstoffsäure und einem Alkylhalogenid.

26. Katalytische Zusammensetzung nach einem der Ansprüche 24 bis 25, in der das Molverhältnis von Aluminiumhalogenid zu Additiv zwischen 0,001 und 1 liegt.

27. Katalytische Zusammensetzung nach einem der Ansprüche 24 bis 25, in der das Molverhältnis von Aluminiumhalogenid zu Additiv zwischen 0,001 und 0,1 liegt.

28. Verfahren zur Alkylierung mindestens eines Isoparaffins mit mindestens einem Olefin in Gegenwart einer katalytischen Zusammensetzung nach mindestens einem der Ansprüche 1 bis 27.

29. Verfahren nach Anspruch 28, wobei das Olefin 2 bis 7 Kohlenstoffatome umfaßt.

30. Verfahren nach einem der Ansprüche 28 bis 30, wobei das Isoparaffin ausgewählt wird aus der Gruppe, die besteht aus Isobutan, 2-Methylbutan, 2-Methyl- und 3-Methyl-pentanen und Isopentan.

31. Verfahren nach einem der Ansprüche 28 bis 30, wobei das Olefin ausgewählt wird aus der Gruppe, die besteht aus Ethylen, Propylen, n-Butenen, Isobuten, n-Pentenen und iso-Pentenen.

32. Verfahren nach einem der Ansprüche 28 bis 31, wobei die Reaktion kontinuierlich oder halbkontinuierlich mit einem Molverhältnis von Paraffin zu Olefin durchgeführt wird, das zwischen 2 und 100 liegt.

33. Verfahren nach einem der Ansprüche 28 bis 32, wobei die Reaktion mit einem Molverhältnis von Paraffin zu Olefin von 5 bis 20 durchgeführt wird.

34. Verfahren nach einem der Ansprüche 28 bis 33, wobei mindestens ein elektrophiles Additiv zugegeben wird.

35. Verfahren nach einem der Ansprüche 28 bis 34, wobei mindestens ein Additiv zugegeben wird, das ausgewählt wird aus der Gruppe, die besteht aus einem Aluminiumhalogenid, einer Halogenwasserstoffsäure und einem Alkylhalogenid.

36. Verfahren nach einem der Ansprüche 34 bis 35, worin das Molverhältnis von Ammoniumhalogenid zu Additiv zwischen 0,001 und 1 liegt.

37. Verfahren nach einem der Ansprüche 34 bis 35, worin das Molverhältnis von Ammoniumhalogenid zu Additiv zwischen 0,001 und 0,1 liegt.

38. Verfahren zur Herstellung einer katalytischen Zusammensetzung nach einem der Ansprüche 1 bis 27, wobei mindestens ein Aluminiumhalogenid, mindestens ein quaternäres Ammoniumhalogenid und/oder mindestens ein Aminhydrohalogenid sowie mindestens eine Kupfer(I)-Verbindung bei einer Temperatur zwischen -20 und +80°C in Gegenwart einer flüssigen polaren organischen Verbindung, die wenig basisch und mit dem Komponenten-Gemisch mischbar ist, miteinander in Kontakt gebracht werden.
